# EUROPÄISCHE PATENTSCHRIFT

(11) **EP 0 377 904 B1**
(45) Veröffentlichungstag und Bekanntmachung des Hinweises auf die Patenterteilung: **18.08.1993**
(21) Anmeldenummer: 89124124.2
(22) Anmeldetag: 28.12.1989
(51) Int. Cl.: A61K 31/53, C07D 253/075

(54) **Mittel gegen Protozoen bei Insekten**
Agent against protozoae in insects
Agent contre les protozoaires chez les insectes

(30) Priorität: 09.01.1989 DE 3900374
(43) Veröffentlichungstag der Anmeldung: 18.07.1990
(73) Patentinhaber: BAYER AG, 51368 Leverkusen (DE)
(72) Erfinder: Mehlhorn, Heinz, Prof.-Dr., D-4040 Neuss (DE); Schmahl, Günter, Dr., D-4630 Bochum (DE); Lindner, Werner, Dr., D-5000 Koeln 80 (DE); Haberkorn, Axel, Prof.-Dr., D-5600 Wuppertal 1 (DE)

(56) Entgegenhaltungen:
- EP-A- 0 170 316
- EP-A- 0 232 932
- EP-A- 0 330 041
- US-A- 3 912 723

## Beschreibung

Die vorliegende Erfindung betrifft Mittel, die 1,2,4-Triazindione enthalten gegen parasitäre Protozoen (Einzeller) bei Insekten.

Zu den Protozoen gehören Parasiten, die bei Insekten weit verbreitet sind (z.B. Nosema apis bei Bienen). Es sind davon vom Menschen gehaltene Nutzinsekten (z.B. Bienen oder Seidenraupen), aber auch im Labor gezüchtete Insekten sowie alle freilebenden Insekten betroffen. Die Parasiten schädigen die Wirtstiere durch Zerstörung ihrer Organe. Zusammen mit anderen Parasiten (z.B. Varroamilben bei Bienen), können sie erhebliche Schädigungen der Wirtstiere hervorrufen, die oft zu deren Tod führen. Bei der Honigbiene rufen sie erheblichen Schaden durch Verminderung der Honigproduktion, Schwächung und Absterben der Völker hervor. Bei Laborzuchten von Insekten, die zur Sammlung genetischen Materials und Erhaltung von Arten gehalten werden, sind oft nur wenige Tiere vorhanden, mit deren Absterben wertvolle Informationen verloren gehen.

Es wurde gefunden, daß die substituierten 1,2,4-Triazindione der allgemeinen Formel (I) 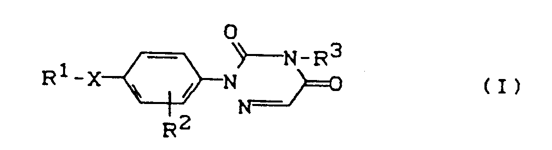
in welcher
- R¹: für aromatische oder über Kohlenstoff gebundene heteroaromatische Reste steht, die gegebenenfalls substituiert sind
- X: für 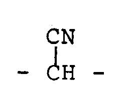 steht,
- R²: für einen oder mehrere, gleiche oder verschiedene Reste der Gruppe Wasserstoff, Halogen, Nitro, Alkyl, Alkoxy, Halogenalkyl, Halogenalkoxy
- R³: für Wasserstoff, gegebenenfalls substituiertes Alkyl, Alkenyl, Alkinyl, Aralkyl steht
sowie ihre Salze mit Basen zur Bekämpfung von Protozoen bei Insekten verwendet werden können.

Die Triazindione sind z. T. bekannt aus EP-OS 170 316. Neue Verbindungen der Formel I lassen sich analog zu den darin angegebenen Herstellmethoden oder analog zu den in EP-OS 330 041 angegebenen Herstellmethoden herstellen.

Bevorzugt verwendet werden Verbindungen der Formel (I), in welcher
- R¹: für gegebenenfalls durch Halogen, Alkyl, Cyano, Nitro, o-Alkyl, s-Alkyl, Halogenalkyl, substituiertes Phenyl, Thiazolyl, Oxazolyl, Benzthiazolyl oder Benzoxazolyl steht.
- X: für 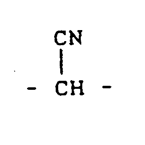 steht
- R²: für Halogen oder C₁₋₆-Alkyl steht
- R³: für Wasserstoff oder C₁-C₄-Alkyl, insbesondere Methyl, steht.

Besonders bevorzugt werden Verbindungen der Formel (I) verwendet, in welcher
- X: für 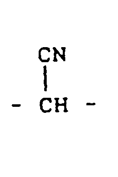 steht,
- R¹: für Thiazolyl, Benzthiazolyl, Benzoxazolyl oder Phenyl,
die gegebenenfalls durch C₁₋₄-Alkyl, insbesondere Methyl, C₁₋₄-Halogenalkyl insbesondere Trifluormethyl, Halogen, insbesondere Chlor, Brom, Fluor, Nitro, CN, C₁₋₄-Alkoxy insbesondere Methoxy, C₁₋₄-Halogenalkoxy insbesondere Trifluormethoxy, C₁₋₄-Alkylthio insbesondere Methylthio, C₁₋₄-Halogenalkylthio insbesondere Trifluormethylthio substituiert sind, steht,
- R²: für einen oder mehrere Reste der Gruppe Wasserstoff oder Halogen insbesondere Chlor, Brom, C₁₋₄-Alkyl insbesondere Methyl steht,
- R³: für Wasserstoff steht.

Besonders bevorzugt werden auch Verbindungen der Formel (I) verwendet, in welcher
- X: für 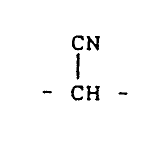 steht
- R¹: für gegebenenfalls durch Chlor, Methyl, Trifluormethyl substituiertes Phenyl steht,
- R²: für einen oder mehrere gleiche oder verschiedene Reste aus der Gruppe Wasserstoff, Chlor, Methyl steht,
- R³: für Wasserstoff oder Methyl steht.

Insbesondere seien genannt:
2-Chlor-α-(4-chlorphenyl)-4-(4,5-dihydro-3,5-dioxo-1,2,4-triazin-2(3H)-yl)-phenylacetonitril (Clazuril) und
2,6-Dichlor-α-(4-chlorphenyl)-4-(4,5-dihydro-3,5-dioxo-1,2,4-triazin-2(3H)-yl)-phenylacetonitril (Diclazuril).

Es war bekannt, daß sich 1,2,4-Triazindione der Formel (I), in welcher
- X: für 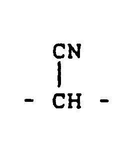 und
- R¹: für Phenyl steht,
zur Bekämpfung von Coccidien der Säugetiere und des Geflügels einsetzen lassen. Auch für die noch nicht bekannten Verbindungen der Formel (I) wird diese Wirkung angegeben. Es war nichts darüber bekannt, daß die Verbindungen der Formel (I) eingesetzt werden können, um Protozoen bei Insekten zu bekämpfen.

Zu den Protozoen die als Parasiten bei Insekten auftreten gehören die Schädlinge des Stamms Microsporidia, insbesondere der Gattung Nosema. Besonders genannt sei Nosema apis, Erreger der Bienenruhr.

Zu den Insekten gehören die vom Menschen gehaltenen Nutz- und Zuchtinsekten, wie z.B. Honigbienen, Seidenraupen, Schlupfwespen aber auch alle in Laborzuchten gehaltenen Insekten die entweder zu Versuchszwecken oder zur Sammlung genetischen Materials gehalten werden.

Die Verbindungen der Formel (I) lassen sich bei allen Entwicklungsstadien der Insekten anwenden.

Die Behandlung kann auch mit Mitteln durchgeführt werden, die neben den angegebenen Wirkstoffen Wirkstoffe gegen andere Schädlinge enthalten. So ist z.B. bei Bienen eine kombinierte Behandlung gegen Nosema und Varroa jacobsoni möglich, wenn die Mittel neben den angegebenen Wirkstoffen, z.B. synthetische Phosphorsäureester wie Coumaphos, Malathion, Formamidine wie Chlordimeform, Phenothiazine wie Promazin, synthetische Pyrethroide wie Flumethrin, Cyfluthrin, Cyhalothrin oder Amitraz oder Cymiazol, enthalten.

Die Behandlung der Protozoen auf den Insekten kann auf verschiedene Weise erfolgen:
1. durch direkten Kontakt mit dem Wirkstoff. Dazu wird dieser z.B. versprüht, verstäubt, verräuchert, verdampft, verdunstet oder in Trägerstoffe, die mit den Insekten in Kontakt kommen, eingearbeitet oder auf die Trägerstoffe aufgebracht,
2. durch eine systemische Wirkung über die Hämolymphe der Insekten. Dazu wird der Wirkstoff z.B. mit dem Futter oder Trinkwasser angeboten, oder bei volkbildenden Insekten in den Stock gegossen, gespritzt oder gesprüht.

Die Behandlung kann prinzipiell ganzjährig erfolgen.

Da Protozoen in der warmen Jahreszeit verstärkt auftreten, ist eine Behandlung zu Beginn der warmen Jahreszeit besonders bevorzugt.

Wird der Wirkstoff verdampft, verdunstet oder ist er in Trägerstoffe eingearbeitet, wird bevorzugt ganzjährig behandelt.

Bei Honigbienen wird die Behandlung besonders vorteilhaft zum Zeitpunkt der Wintereinfütterung oder in der brutfreien Zeit durchgeführt.

Weiter kann bei Honigbienen das Bienenvolk als Kunstschwarm behandelt werden. Dies kann auch während der Brutperiode erfolgen.

Mittel, die versprüht werden, enthalten den Wirkstoff in Konzentrationen von 0,1 - 50 Gew.-%, bevorzugt von 0,3 - 20 Gew.-%.

Die Mittel können vor der Anwendung auf Wirkstoff-Konzentrationen von 10⁻⁴ - 2 Gew.-%, bevorzugt 10⁻³ - 0,5 Gew.-%, verdünnt werden. Sie werden konventionell mit üblichen Geräten versprüht.

Mit diesen Mitteln werden entweder die Insekten oder ihre Wohnung oder Teile davon oder ihre Umgebung behandelt.

Die Mittel enthalten den Wirkstoff neben Verdünnungsmitteln und/oder Emulgatoren, die in den angewendeten Konzentrationen insektenverträglich sind.

Geeignete Verdünnungsmittel sind Wasser, Alkohole, wie Methanol, Ethylalkohol, Propanol, Isopropylalkohol, n-Butylalkohol, Amylalkohol, Octanol;
Glykole, wie Propylenglykol, 1,3-Butylenglykol, Ethylenglykol, Dipropylenglykolmonomethylether; Diethylenglykolmonomethylether;
Glycerin;
aromatische Alkohole wie Benzylalkohol;
Carbonsäureester, wie z. B. Ethylacetat, Benzylbenzoat, Butylacetat, Propylencarbonat, Milchsäureethylester;
aliphatische Kohlenwasserstoffe, Öle, wie z. B. Baumwollsaatöl, Erdnußöl, Maiskeimöl, Olivenöl, Ricinusöl, Sesamöl;
Ketone, wie z. B. Aceton und Methylethylketon;
synth. Mono- und Triglyceride mit natürlichen Fettsäuren.

Weiterhin sind u. a. Verbindungen wie Dimethylsulfoxid, Dimethylacetamid, Dimethylformamid, N-Methylpyrrolidon, Dioxan, 2-Dimethyl-4-oxymethyl-1,3-dioxolan gut als Verdünnungsmittel geeignet.

Besonders geeignet sind Wasser sowie niedere Alkohole mit bis zu 8 Kohlenstoffatomen im Molekül, sowie niedere Ketone wie Methylethylketon und Ether des Ethylenglykols und des Propylenglykols.

Es können bei der Herstellung der erfindungsgemäß verwendbaren Mittel ein oder mehrere Verdünnungsmittel eingesetzt werden.

Geeignete Emulgatoren sind:
anionaktive Tenside, wie Na-Laurylsulfat, Fettalkoholethersulfate, Mono/Dialkylpolyglykoletherorthophosphorsäureester-Monoethanolaminsalze, Calciumalkylarylsulfonat,
kationaktive Tenside, wie Cetyltrimethylammoniumchlorid,
ampholytische Tenside, wie Di-Na-N-lauryl-β-iminodipropionat oder Lecithin,
nicht ionogene Tenside, z. B. polyoxyethyliertes Ricinusöl, polyoxyethyliertes Sorbitan-Monooleat oder Sorbitan-Monostearat, Glycerinmonostearat, Polyoxyethylenstearat, Alkylphenolpolyglykolether.

Bevorzugt seien als Emulgatoren genannt:
nicht-ionische, wasserlösliche Emulgatoren mit einem HLB (hydrophilic/lipophilic-balance-Wert) größer als 10, z.B. Emulgator NP 10® (Bayer AG), Alkylarylpolyglykolether; Renex 678® (Atlas Chemical Industries), Polyoxyethylenalkylarylether; Tween 40® (Atlas), Polyoxyethylensorbitanmonopalmitat; Myri 53® (Atlas), Polyoxyethylenstearat; Atlas G 3707®, Polyoxyethylenlaurylether; Atlas G 3920®, Polyoxyethylenoleylether; Atlas G 9046 T®, Polyoxyethylenmannitanmonolaurat; Emulgator 1371 B® (Bayer AG), Alkylpolyglykolether; Emulgator 1736® (Bayer AG), Alkylpolyglykolether (Oleylpolyglykolether); Emulgator OX® (Bayer AG), Alkylpolyglykolether (Dodecylpolyglykolether); Ninox BM-2® (Stepan Chemical Co.), ethoxyliertes Nonylphenol; Triton X-100® (Rohm und Haas Co.), Isooctylphenolpolyethoxyethanol; Cremophor EL®, polyoxyethyliertes Ricinusöl.

Die Emulgatoren sind in den erfindungsgemäßen Mitteln in Konzentrationen vom bis zu 10-fachen, bevorzugt bis zu 5-fachen, des eingesetzten Wirkstoffs enthalten. Die Verdünnungsmittel werden jeweils zur gewünschten Endkonzentration ergänzt.

Die Mittel werden hergestellt durch Lösen des oder der Wirkstoffe im Lösungsmittel oder in einem Emulgator oder Emulgator-Lösungsmittelgemisch, falls notwendig, unter Erwärmung. Erforderlichenfalls erfolgt eine weitere Verdünnung der Mittel mit Wasser auf die gewünschte Konzentration.

Die Mittel zum Stäuben enthalten den Wirkstoff neben herkömmlichen insektenverträglichen Trägerstoffen, die zur Herstellung von Pudern oder wettable-powder geeignet sind.

Geeignete Trägerstoffe sind anorganische Trägerstoffe wie z. B. Talkum, Kaolin, Calciumcarbonat, Silikate, Bentonite, ferner organische Trägerstoffe wie z. B. Stärken, z. B. Reisstärke, Zucker, Zellulose und Zellulosederivate.
Die Mittel werden gegebenenfalls unter Zusatz von Netzmitteln durch Vermischen des oder der Wirkstoffe mit den Trägerstoffen hergestellt. Geeignete Netzmittel sind z.B. die weiter oben angeführten Emulgatoren.

Mittel, die verräuchert werden, enthalten den Wirkstoff in Konzentrationen von 10⁻⁷ - 2 Gew.-% pro 100 g Trägermaterial. Als Trägermaterial dient das übliche Material für Räucherpräparate.

Mittel, durch die der Wirkstoff verdampft wird, sind z.B. Trägermaterialien, die mit wirkstoffhaltigen Mitteln imprägniert sind oder in die der Wirkstoff eingearbeitet ist. Bevorzugt sind mit wirkstoffhaltigen Mitteln getränkte Papier-, Pappe-, Zellulose-, Stoff-, Filz-, Flies-, Lederplättchen oder -folien, die z.B. durch eine Wärmequelle erhitzt werden können. Als Wärmequelle seien die für Verdampferplättchen üblichen elektrischen bzw. batteriebetriebenen Verdampferöfchen genannt.

Besonders vorteilhaft ist es, Mittel einzusetzen, in die der Wirkstoff eingearbeitet oder auf die er aufgebracht ist und die ohne zusätzliche Wärmequelle wirken. Damit läßt sich die Behandlung besonders einfach durchführen. Die Mittel werden einfach in die Wohnung der Insekten eingeführt.

Die Behandlung wird durch Entfernung des Mittels beendet. Dies verhindert, daß die Parasiten ständig abnehmenden Wirkstoffkonzentrationen ausgesetzt sind. Dadurch wird einer Resistenzbildung bei den Parasiten vorgebeugt.

Die lang anhaltende Wirkstoffabgabe dieser Mittel erlaubt eine Langzeittherapie, die auch den Nachwuchs aus der Brut der Insekten erfaßt.

Der Wirkstoff kann bei diesen Mitteln in Trägerstoffen enthalten oder eingearbeitet sein oder in geeigneter Form auf Trägerstoffen aufgebracht sein.

Trägerstoffe sind Formkörper, die am oder in der Insektenwohnung angebracht werden. Auch Teile der Wohnung können aus Material geformt werden, in das der Wirkstoff eingearbeitet ist oder auf dessen Oberfläche der Wirkstoff aufgebracht ist oder das mit Wirkstoff getränkt oder imprägniert ist. Bevorzugt sind z.B. bei Bienen Schiede, die zwischen die Waben eingeschoben werden und die mit wirkstoffhaltigen Mitteln behandelt worden sind oder in die der Wirkstoff eingearbeitet ist.

Als Trägerstoffe können natürliche oder synthetische Trägerstoffe dienen. Natürliche Trägerstoffe sind z.B. Holz, Holzverarbeitungsprodukte, Pappe, Papier, Gummi, Kautschuk, Filz, Metall, Glas, Porzellan, keramische Materialien. Synthetische Trägerstoffe sind z.B. Kunststoffe auf Basis Polyvinyl, PVC, Polyacrylat, Polymethacrylat, Epoxid, Polyurethan, Polyester, Polyamid, Cellulose und deren Derivate, Polyethylen, Polypropylen, synthetischer Kautschuk.

Als Trägerstoffe kommen aber auch Schichten in Frage, die auf einen festen oder flexiblen Untergrund aufgebracht sind. Solche Schichten können saugfähig sein und mit wirkstoffhaltigen Mitteln behandelt werden. Sie können aber auch nicht saugfähig sein und den Wirkstoff eingearbeitet enthalten. In der Regel handelt es sich bei diesen Schichten um haftfähige Polymere, denen gegebenenfalls inerte Füllsubstanzen zugesetzt werden. Als Polymere werden dabei die Lackrohstoffe der Farbenindustrie sowie z. B. Cellulosederivate, Acrylate und Methacrylate eingesetzt.

Als Beispiele für Füllstoffe zur Herstellung saugfähiger Schichten seien genannt: Kaolin, Calciumcarbonat, Silikate, Bentonite, Cellulose, Cellulosederivate, Stärke, Holzpulver. Der Wirkstoff ist dabei entweder bereits in schichtbildendes Material eingearbeitet oder die Schicht wird nachträglich z. B. mit dem oben beschriebenen zu versprühenden Mittel getränkt oder imprägniert oder besprüht.

Schichten, die den Wirkstoff eingearbeitet enthalten, können auch durch wirkstoffhaltige Anstriche oder Lacke gebildet werden. Diese enthalten den Wirkstoff in einer Konzentration von 0,00001 - 10, bevorzugt 0,001- 1 Gewichtsprozent neben der üblichen Beschichtungsgrundmasse. Bevorzugt werden Dispersionsanstriche und -lacke als Beschichtungsgrundmasse eingesetzt.

Schichten, die den Wirkstoff eingearbeitet enthalten, können aber auch Folien, Streifen, Bänder sein, die ein-oder mehrschichtig, sowie gegebenenfalls selbstklebend ausgebildet sind.

So kann eine wirkstoffhaltige Selbstklebefolie z. B. aus einer Klebeschicht, einer flexiblen Trägerschicht, einer wirkstoffhaltigen flexiblen Trägerschicht, einer wirkstofffreien flexiblen Deckschicht bestehen. Die einzelnen Schichten bestehen aus an sich bekannten Polymermaterialien, die für die Herstellung solcher Schichten geeignet sind.

Wie bereits erwähnt, kann der Wirkstoff in diesen Formkörpern eingearbeitet enthalten sein. Diese enthalten den Wirkstoff in Konzentrationen von 0,00001 - 10 Gew.-%, bevorzugt 0,00001 - 1 Gew.-%, bezogen auf das Grundmaterial des Formkörpers.

Geeignete Formkörper sind Streifen, Bänder, Platten, aber auch, wie weiter oben erwähnt, Bauteile.

Für die Herstellung der erfindungsgemäßen Formkörper können Polyvinylharze, Polyacrylate, Epoxyharze, Cellulose, Cellulosederivate, Polyamide und Polyester verwendet werden, die mit den obengenannten Wirkstoffen ausreichend verträglich sind. Die Polymeren müssen eine ausreichende Festigkeit und Biegsamkeit haben, um beim Formen nicht zu reißen oder brüchig zu werden. Sie müssen eine ausreichende Wanderung der Wirkstoffe an die Oberfläche des Formkörpers zulassen.

Typische Vinylharze sind beispielsweise Polyvinylhalogenide, wie Polyvinylchlorid, Polyvinylchlorid-Vinylacetat und Polyvinylfluorid; Polyacrylat- und Polymethacrylatester, wie Polymethylacrylat und Polymethylmethacrylat; und Polyvinylbenzole, wie Polystyrol und Polyvinyltoluol.

Für die Herstellung der erfindungsgemäßen Formkörper auf der Basis Polyvinylharz sind die Weichmacher geeignet, die üblicherweise zum Weichmachen von festen Vinylharzen verwendet werden. Der verwendete Weichmacher hängt von dem Harz und seiner Verträglichkeit mit dem Weichmacher ab. Geeignete Weichmacher sind beispielsweise Ester von Phosphorsäure, wie Tricresylphosphat, Ester von Phthalsäure, wie Dimethylphthalat und Dioctylphthalat, und Ester von Adipinsäure, wie Diisobutyladipat. Es können auch andere Ester, wie die Ester von Azelainsäure, Maleinsäure, Ricinolsäure, Myristinsäure, Palmitinsäure, Ölsäure, Sebacinsäure, Stearinsäure und Trimellithsäure, sowie komplexe lineare Polyester, polymere Weichmacher und epoxydierte Sojabohnenöle verwendet werden. Die Menge des Weichmachers beträgt etwa 10 bis 50 Gew.-%, vorzugsweise etwa 20 bis 45 Gew.-% der gesamten Zusammensetzung.

In den Formkörpern können noch weitere Bestandteile, wie Stabilisierungsmittel, Schmiermittel, Füllstoffe und Färbematerialien, enthalten sein, ohne daß dadurch die grundlegenden Eigenschaften der Zusammensetzung verändert werden. Geeignete Stabilisierungsmittel sind Antioxydationsmittel und Mittel, die den Formkörper vor ultravioletter Strahlung und unerwünschtem Abbau während der Bearbeitung, wie Strangpressen schützen. Einige Netzmittel wie epoxydierte Sojabohnenöle, dienen außerdem als sekundäre Weichmacher. Als Schmiermittel können beispielsweise Stearate, Stearinsäure und Polyethylen mit niedrigem Molekulargewicht verwendet werden. Diese Bestandteile können in einer Konzentration bis zu etwa 20 Gew.-% der gesamten Zusammensetzung verwendet werden.

Bei der Herstellung der erfindungsgemäßen Formkörper auf Vinylharzbasis werden die verschiedenen Bestandteile nach bekannten Mischverfahren trocken gemischt und nach bekannten Strangpreß- oder Spritzgußverfahren formgepreßt.

Die Wahl des Verarbeitungsverfahrens zur Herstellung der erfindungsgemäßen Formkörper richtet sich technisch grundsätzlich nach den rheologischen Eigenschaften des Formkörpermateriales und der Form des gewünschten Gebildes. Die Verarbeitungsverfahren können nach der Verarbeitungstechnologie oder nach der Art der Formgebung eingestellt werden. Bei der Verfahrenstechnologie kann man die Verfahren nach den bei ihnen durchlaufenen rheologischen Zuständen unterteilen. Danach kommen für viskose Formkörpermaterialien Gießen, Pressen, Spritzen und Auftragen und für elastoviskose Polymere Spritzgießen, Strangpressen (Extrudieren), Kalandrieren, Walzen und gegebenenfalls Kanten in Frage. Nach Art der Formgebung eingeteilt, lassen sich die erfindungsgemäßen Formkörper durch Gießen, Tauchen, Pressen, Spritzgießen, Extrudieren, Kalandrieren, Prägen, Biegen, Tiefziehen etc. herstellen.

Diese Verarbeitungsverfahren sind bekannt und bedürfen keiner näheren Erklärung. Im Prinzip gelten für Polymere wie Polyamide und Polyester die Erläuterungen, die oben beispielhaft für Polyvinylharze gemacht wurden.

Mittel, die durch eine systemische Wirkung über die Hämolymphe der Insekten wirken, sind z.B. Futterstoffe die die Wirkstoffe enthalten. Als solche seien genannt: Zuckergranulate, zuckerhaltige Mischungen, Lösungen, Suspensionen oder Emulsionen. Diese enthalten Wirkstoffkonzentrationen von 0,5 - 20 Gew.-%, vorzugsweise von 1 - 10 Gew.-%. Diese Mischungen werden mit Wasser oder Zuckerlösung auf Anwendungskonzentrationen des Wirkstaffs von 10⁻⁸ - 1 Gew.-%, bevorzugt 0,0001 - 0,01 Gew.-%, besonders bevorzugt auf 0,0001 - 0,005 Gew.-% weiter verdünnt.

Ferner seien genannt anwendungsfertige Futterteige oder Knete, die den Wirkstoff in der Anwendungskonzentration neben Zucker und Stärke enthalten.

Besonders bevorzugt sind Mittel für eine Anwendung der Wirkstoffe im Trinkwasser. Dafür kommen wassermischbare Lösungen der Wirkstoffe, die ein oder mehrere polare Lösungsmittel enthalten und alkalisch reagieren, in Frage.

Zur Herstellung solcher Lösungen wird der Wirkstoff in einem polaren, wasserlöslichen Lösungsmittel gelöst, welches entweder alkalisch reagiert oder dem eine alkalische wasserlösliche Substanz zugefügt wird. Letztere wird zweckmäßigerweise ebenfalls im Lösungsmittel gelöst, kann aber auch in dem Lösungsmittel suspendiert sein und sich erst im Trinkwasser lösen. Dabei soll das Trinkwasser nach Zusatz der Wirkstofflösung einen pH-Wert von mehr als 7, vorzugsweise aber einen pH-Wert größer als pH 8 und kleiner als 11 haben.

Das Trinkwasser kann einen Zuckergehalt (Glukose) von 0,1 bis 5 Gew.-%, bevorzugt von ca. 1 Gew.-% haben.

Die Lösung des Wirkstoffkonzentrats sollte einen pH von 11 nicht überschreiten.

Die Konzentration des Wirkstoffs kann im Bereich von 0,5 bis 50 % liegen, vorzugsweise aber in einem Bereich von 1 bis 25 %.

Als Lösungsmittel kommen alle wasserlöslichen Lösungsmittel in Betracht, in denen der Wirkstoff in genügender Konzentration löslich ist und die physiologisch unbedenklich sind.

Dies sind aus der Reihe der Alkohole ein- und mehrwertige wie z.B. Ethylalkohol, Isopropylalkohol, Benzylalkohol, Glycerin, Propylenglykol, Polyethylenglykole, Poly(oxyethylen)-poly(oxypropylen)-Polymere, basische Alkohole wie z.B. Mono-, Di- und Triethanolamin.

Außerdem sind geeignet Ketone z.B. Aceton oder Methylethylketon und aus der Reihe der Ester z.B. Milchsäureethylester. Andere Lösungsmittel wie N-Methylpyrrolidon, Dimethylacetamid, Dimethylformamid können ebenfalls eingesetzt werden.

Als Basen zur Einstellung des alkalischen pH-Wertes sind vorzugsweise organische Basen einzusetzen, z.B. basische Aminosäuren wie L- bzw. D,L-Arginin, L- bzw. D,L-Lysin, Methylglucosamin, Glucosamin, 2-Amino-2-hydroxymethylpropandiol-(1,3), Cholin, Piperazin. Auch Diamine sind hier geeignet z.B. bildet N,N,N′,N′-Tetrakis-(2-hydroxypropyl)-ethylen-diamin oder Polyether-tetrol auf der Basis Ethylendiamin (M.G. 480-420, OH-Index 432-467) ebenfalls klare Lösungen im angegebenen pH-Bereich aus. Auch anorganische Basen können eingesetzt werden, z.B. Ammoniak oder Natriumcarbonat - gegebenenfalls unter Zugabe von Wasser.

Substanzen, die sonst als Emulgatoren oder Solubilisatoren verwendet werden und in Wasser kolloidal löslich sind, können in diesem Falle wie polare Lösungsmittel eingesetzt werden, sofern ihnen noch ein basischer Hilfsstoff zugemischt wird.

Zur Herstellung der Lösungen werden die Substanzen in einen Behälter mit Rührwerk eingewogen und dann unter Erwärmen solange gerührt, bis eine klare Lösung entstanden ist.

Zubereitungen Wirkstoffe sind z.B.:

### Beispiel 1

| | |
|---|---|
| Wirkstoff gemäß Formel I | 5,0 g |
| Propylenglykol | 50,0 g |
| Natriumcarbonat | 5,0 g |
| Wasser ad | 100 ml |

### Beispiel 2

| | |
|---|---|
| Wirkstoff gemäß Formel I | 5,0 g |
| Monoethanolamin | 10 g |
| N-Methylpyrrolidon ad | 100 ml |

### Beispiel 3

| | |
|---|---|
| Wirkstoff gemäß Formel I | 2,5 g |
| Natriumcarbonat | 5,0 g |
| Polyethylenglykol200 ad | 100 ml |

Der Wirkstoff wird unter Erwärmen im Polyethylenglykol gelöst und Natriumcarbonat darin suspendiert.

## Patentansprüche

1. Verwendung von substituierten 1,2,4-Triazindionen der allgemeinen Formel (I) 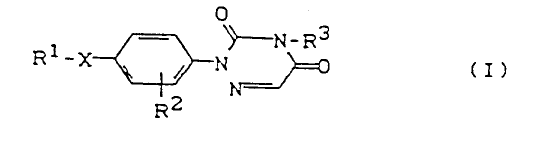 in welcher
R¹ für aromatische oder über Kohlenstoff gebundene heteroaromatische Reste steht, die gegebenenfalls substituiert sind
X für 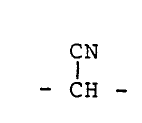 steht,
R² für einen oder mehrere, gleiche oder verschiedene Reste der Gruppe Wasserstoff, Halogen, Nitro, Alkyl, Alkoxy, Halogenalkyl, Halogenalkoxy
R³ für Wasserstoff, gegebenenfalls substituiertes Alkyl, Alkenyl, Alkinyl, Aralkyl steht
sowie ihrer Salze mit Basen zur Herstellung von Arzneimitteln zur Bekämpfung von Protozoen bei Insekten.

2. Verwendung gemäß Anspruch 1 von Verbindungen der Formel (I), in welcher
R¹ für gegebenenfalls durch Halogen, Alkyl, Cyano, Nitro, o-Alkyl, s-Alkyl, Halogenalkyl, substituiertes Phenyl, Thiazolyl, Oxazolyl, Benzthiazolyl oder Benzoxazolyl steht.
X für 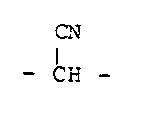 steht
R² für Halogen oder C₁₋₆-Alkyl steht
R³ für Wasserstoff oder C₁₋₄-Alkyl steht.

3. Verwendung gemäß Anspruch 1 von Verbindungen der Formel (I), in welcher
X für 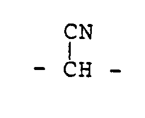 steht,
R¹ für Thiazolyl, Benzthiazolyl, Benzoxazolyl oder Phenyl,
die gegebenenfalls durch C₁₋₄-Alkyl, insbesondere Methyl, C₁₋₄-Halogenalkyl insbesondere Trifluormethyl, Halogen, insbesondere Chlor, Brom, Fluor, Nitro, CN, C₁₋₄-Alkoxy insbesondere Methoxy, C₁₋₄-Halogenalkoxy insbesondere Trifluormethoxy, C₁₋₄-Alkylthio insbesondere Methylthio, C₁₋₄-Halogenalkylthio insbesondere Trifluormethylthio substituiert sind, steht,
R² für einen oder mehrere Reste der Gruppe Wasserstoff oder Halogen insbesondere Chlor, Brom, C₁₋₄-Alkyl insbesondere Methyl steht,
R³ für Wasserstoff steht.

4. Verwendung gemäß Anspruch 1 von Verbindungen der Formel (I), in welcher
X für 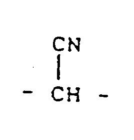 steht
R¹ für gegebenenfalls durch Chlor, Methyl, Trifluormethyl substituiertes Phenyl steht,
R² für einen oder mehrere gleiche oder verschiedene Reste aus der Gruppe Wasserstoff, Chlor, Methyl steht,
R³ für Wasserstoff oder Methyl steht.

5. Verwendung gemäß Anspruch 1 von
2-Chlor-α-(4-chlorphenyl)-4-(4,5-dihydro-3,5-dioxo-1,2,4-triazin-2(3H)-yl)-phenylacetonitril (Clazuril) und
2,6-Dichlor-α-(4-chlorphenyl)-4-(4,5-dihydro-3,5-dioxo-1,2,4-triazin-2(3H)-yl)-phenylacetonitril (Diclazuril).

## Claims

1. Use of substituted 1,2,4-triazinedions of the general formula (I) 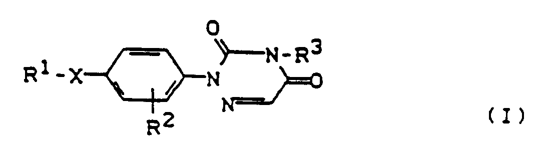 in which
R¹ represents optionally substituted aromatic radicals or optionally substituted heteroaromatic radicals which are bonded via carbon,
X represents 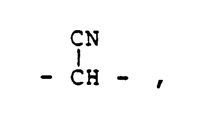
R² represents one or more identical or different radicals from the group comprising hydrogen, halogen, nitro, alkyl, alkoxy, halogenoalkyl and halogenoalkoxy,
R³ represents hydrogen or optionally substituted alkyl, alkenyl, alkynyl or aralkyl,
as well as their salts with bases for the preparation of medicaments for combatting Protozoa in insects.

2. Use according to Claim 1 of compounds of the formula (I) in which
R¹ represents phenyl, thiazolyl, oxazolyl, benzothiazolyl or benzoxazolyl, each of which is optionally substituted by halogen, alkyl, cyano, nitro, o-alkyl, s-alkyl or halogenoalkyl,
X represents 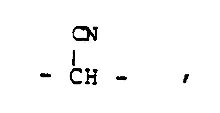
R² represents halogen or C₁₋₆-alkyl and
R³ represents hydrogen or C₁₋₄-alkyl.

3. Use according to Claim 1 of compounds of the formula (I) in which
X represents 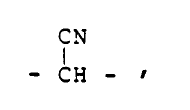
R¹ represents thiazolyl, benzothiazolyl, benzoxazolyl or phenyl,
each of which is optionally substituted by C₁₋₄-alkyl, in particular methyl, C₁₋₄-halogenoalkyl, in particular trifluoromethyl, halogen, in particular chlorine, bromine or fluorine, nitro, CN, C₁₋₄-alkoxy, in particular methoxy, C₁₋₄-halogenoalkoxy, in particular trifluoromethoxy, C₁₋₄-alkylthio, in particular methylthio or C₁₋₄-halogenoalkylthio, in particular trifluoromethylthio,
R² represents one or more radicals from the group comprising hydrogen or halogen, in particular chlorine or bromine, or C₁₋₄-alkyl, in particular methyl, and
R³ represents hydrogen.

4. Use according to Claim 1 of compounds of the formula (I) in which
X represents 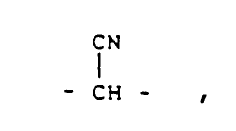
R¹ represents phenyl which is optionally substituted by chlorine, methyl or trifluoromethyl,
R² represents one or more identical or different radicals from the group comprising hydrogen, chlorine and methyl, and
R³ represents hydrogen or methyl.

5. Use according to Claim 1 of 2-chloro-α-(4-chlorophenyl)-4-(4,5-dihydro-3,5-dioxo-1,2,4-triazin-2(3H)-yl)-phenylacetonitrile (clazuril) and 2,6-dichloro-α-(4-chlorophenyl)-4-(4,5-dihydro-3,5-dioxo-1,2,4-triazin- 2(3H)-yl-phenylacetonitrile (diclazuril).

## Revendications

1. Utilisation de 1,2,4-triazinediones substituées de formule générale (I) 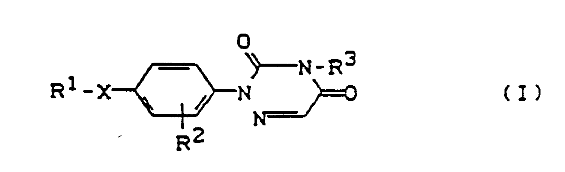 dans laquelle
R¹ signifie des restes aromatiques ou des restes hétéroaromatiques éventuellement substitués fixés par le carbone,
X signifie 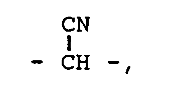
R² signifie un ou plusieurs restes identiques ou différents choisis parmi le groupe comportant l'hydrogène, un halogène, un groupe nitro, alkyle, alcoxy, halogénoalkyle, halogénoalcoxy,
R³ signifie l'hydrogène, un groupe alkyle, alcényle, alcinyle, aralkyle éventuellement substitués, ainsi que leurs sels avec des bases pour fabriquer des médicaments pour la lutte contre les protozoaires chez les insectes.

2. Utilisation selon la revendication 1 de composés de formule (I) dans laquelle
R¹ signifie un reste phényle, thiazolyle, oxazolyle, benzothiazolyle ou benzoxazolyle éventuellement substitués par un halogène, un groupe alkyle, un groupe cyano, un groupe nitro, o-alkyle, s-alkyle, halogénoalkyle;
X signifie 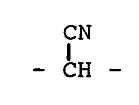
R² signifie un halogène ou un groupe alkyle en C₁₋₆,
R³ signifie l'hydrogène ou un groupe alkyle en C₁₋₄.

3. Utilisation selon la revendication 1 de composés de formule (I), dans laquelle
X signifie 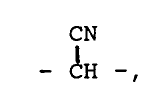
R¹ signifie un groupe thiazolyle, benzothiazolyle, benzoxazolyle ou phényle,
éventuellement substitué par un groupe alkyle en C₁₋₄, en particulier méthyle, halogénoalkyle en C₁₋₄, en particulier trifluorométhyle, un halogène, en particulier le chlore, le brome, le fluor, un groupe nitro, CN, alcoxy en C₁₋₄, en particulier méthoxy, halogénoalcoxy en C₁₋₄, en particulier trifluorométhoxy, un groupe alkylthio en C₁₋₄, en particulier méthylthio, un groupe halogénoalkylthio en C₁₋₄, en particulier trifluorométhylthio,
R² signifie un ou plusieurs restes choisis parmi le groupe comportant l'hydrogène ou un halogène, en particulier le chlore, le brome, un groupe alkyle en C₁₋₄, en particulier méthyle,
R³ signifie l'hydrogène.

4. Utilisation selon la revendication 1, de composés de formule (I), dans laquelle
X signifie 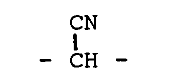
R¹ signifie un groupe phényle éventuellement substitué par le chlore, un groupe méthyle, un groupe trifluorométhyle,
R² signifie un ou plusieurs restes identiques ou différents choisis dans le groupe comportant l'hydrogène, le chlore, un groupe méthyle,
R³ signifie l'hydrogène ou un groupe méthyle.

5. Utilisation selon la revendication 1, de 2-chloro-α-(4-chlorophényl)-4-(4,5-dihydro-3,5-dioxo-1,2,4-triazine-2(3H)-yl)-phénylacétonitrile (Clazuril) et 2,6-dichloro-α-(4-chlorophényl)-4-(4,5-dihydro-3,5-dioxo-1,2,4-triazine-2(3H)-yl)-phénylacétonitrile (Diclazuril).
